# EUROPEAN PATENT SPECIFICATION

(11) **EP 1 368 084 B1**
(45) Date of publication and mention of the grant of the patent: **21.09.2005**
(21) Application number: 02706802.2
(22) Date of filing: 15.03.2002
(51) Int. Cl.: A61M 15/00

(54) **POWDER INHALER**
PULVERINHALATOR
INHALATEUR DE POUDRE

(30) Priority: 16.03.2001 FI 20010538
(43) Date of publication of application: 10.12.2003
(73) Proprietor: Orion Corporation, 02200 Espoo (FI)
(72) Inventor: HAIKARAINEN, Jussi, FIN-02940 Espoo (FI); PURMA, Kalle, FIN-37830 Viiala (FI)
(74) Representative: Merryweather, Colin Henry
(86) International application number: PCT/FI2002/000211
(87) International publication number: WO 2002/074357

(56) References cited:
- EP-A- 0 979 661
- WO-A-93/03782
- WO-A-93/25258
- US-A- 5 575 280

## Description

### Background of the invention

The present invention relates to a device for dispensing of a powdered drug preparation by inhalation. The device is in particular a multidose device in which medicament powder is held in a container, and which is equipped with a metering means, which dispenses doses from the powder container. The device of the invention is useful, for example, in the treatment of asthma.

Multidose type dry powder inhalers comprising a medicament container and a metering member for measuring and dispensing a unit dose are known, for example from patent publications GB 2165159, EP 79478, EP 166294, WO 92/00771 and WO 92/09322. In these devices, a series of dosing recesses are notched into the surface of a cylindrical or a conical metering member. When the metering member is rotated, the dosing recesses in turn will move first to a position in alignment with the powder container for being filled with a dose of powder falling from the powder container. Thereafter the filled dosing recess is moved to a position in alignment with the inhalation channel.

The above devices have a drawback of dosing inaccuracy. The dosing recess is filled with powder when the dosing recess is in alignment with the bottom aperture of the container the powder flowing through the aperture under the effect of gravity. If the aperture is temporarily blocked, e.g. by lumps or by arching of the powder in the container, the dosing recess will be filled incompletely or will remain empty. The reservoir has to be often provided with a significant surplus of powder in order to guarantee a critical mass of powder which is needed for a complete filling of the dosing recess by gravity. Therefore, a significant amount of unused powder will be left in the device after the maximum allowed number of dosing cycles have been performed. Moreover, the dosing accuracy can be harmfully affected by the electrical charges present in the powder particles.

An attempt to improve the-dosing accuracy in a dy powder inhaler of the above type is described in a patent publication WO 96/08284. The device is provided with a moveable weight adapted, when the device is shaken, to strike an anvil surface of the device. This causes the powder in the reservoir to be urged downwardly and is believed to improve the filling of the dosing recess.

EP-A-0,979,661 discusses an inhaler with the features of the pre-characterising portion of claim 1. The inhaler includes a movable weight which strikes an anvil surface when the inhaler is shaken.

It is an object of the invention to provide a powder inhaler, in which the filling of the dosing recesses is still more effective.

### Summary of the invention

The present invention provides a multidose powder inhaler for dispensing powdered medicament by inhalation comprising a medicament container adapted to receive a plurality of powdered medicament doses; an air channel through which air is drawn via a mouthpiece; a metering member for metering a metered dose of the powdered medicament from the medicament container; and a loose agitation element disposed inside the medicament container.

In another aspect, the invention provides a multidose powder inhaler for dispensing powdered medicament by inhalation comprising a medicament container adapted to receive a plurality of powdered medicament doses, an air channel through which air is drawn via a mouthpiece, a metering member having one or more dosing recesses for metering in one position a metered dose of the powdered medicament from the medicament container and for bringing it in another position to the air channel, and a loose agitation element disposed inside the medicament container.

It has been found that a freely movable agitation element, particularly in the form of an agitation sphere, inside the medicament container can put the necessary surplus weight on the powder, when the device is shaken, such that the filling is complete. Consequently, the amount of surplus powder in the medicament container can be reduced and less medicament powder will be wasted. The agitation element also improves the agitation of the powder, when the device is shaken, and thereby maintains the flowability of the powder. Moreover, the agitation element is able to remove local electric charges produced in the powder thus further improving the dosing accuracy. The removal of charges is most effective if the agitation element is manufactured from a conductive material, e.g. a metal.

The agitation element is particularly useful in multidose dry powder inhalers, which have a powder container, particularly cylindrical in shape, and in which the dosing recess is filled under the effect of gravity as the powder flows from the reservoir into the filling recess.

### Brief description of the drawings

FIG. 1 is a cross-sectional side view of the device according to one embodiment of the invention.
FIG. 2 is a cross-sectional side view of the device according to another embodiment of the invention.

### Detailed description of the invention

The present invention provides a multidose powder inhaler, more specifically a multidose dry powder inhaler, for dispensing powdered medicament by inhalation comprising a medicament container adapted to receive a plurality of powdered medicament doses, an air channel through which air is drawn via a mouthpiece, a metering member for metering in one position a metered dose of the powdered medicament from the medicament container and for bringing it in another position to the air channel, and a loose agitation element disposed inside the medicament container.

The medicament container is preferably cylindrical in form and has a certain supply of medicament, e.g. 200 doses. Typically, the medicament container has a powder outlet in the form of an opening at the lower end. The metering member is manually movable and can be in any suitable form. At least one dosing recess in formed in the face of the metering member for receiving in one position a metered dose of the powdered medicament from medicament container and for bringing in another position the metered dose of the powdered medicament to the air channel. Several forms of metering members for multi-dose powder inhalers are known in the art, e.g. a rotatable dosing drum as described in e.g. WO 92/00771 and WO 92/09322, or a movable dosing slide as described in e.g. WO 95/31237 and WO 97/17097. The face of the metering member is adapted to be in contact with a similar mating face at the lower end of the medicament container. A dose of medicament powder is metered from the container, when the outlet of the container and the dosing recess of the metering member are in alignment and the powder flows from the container into the dosing recess.

Preferably the metering member is in the form of a drum or a slide. However, also other forms of metering members can be used in the device of the invention.

The loose agitation element is preferably in the form of an agitation sphere. Other forms are possible such as cylindrical or disc-like forms. However a sphere is preferred. The agitation sphere can be completely rigid or hollow. The surface of the sphere can be smooth, however it may be advantageous to provide the sphere with a rough surface.

The diameter of the agitation sphere is selected such that it can freely move within the medicament container, when the user shakes the powder inhaler in the flow direction of the powder, typically in generally upward and downward motion. Thus, the diameter of the agitation sphere is preferably 10 - 90 %, more preferably 30 - 80 %, from the diameter of the medicament container.

Alternatively, the medicament container may contain a plurality of loose agitation spheres. For example, the agitation element may consist of three agitation spheres having equivalent diameter.

The weight of the agitation sphere is selected such that it is sufficient to effectively put weight on the powder and press the powder towards the dosing recess, when the device is shaken. However, the sphere should not be too heavy, since harmful packing of the powder may then occur.

The agitation sphere can be prepared from any suitable rigid and durable material, for example plastics, resins or metals such as stainless steel. A conductive material, such as metal orconductive polymer, is particularly preferred, since it is able to remove harmful local static charges produced in the powder. It is particularly preferred if the local charges present in the powder can be transferred via the agitation sphere out of the container. This can be accomplished e.g. by providing the lid of the container with a conductive material leading to the operating member of the device such as a depressible cover. Thereby the charges are removed from the container as the agitating sphere hits the conductive lid, when the device is shaken, and from the lid to the finger of the user.

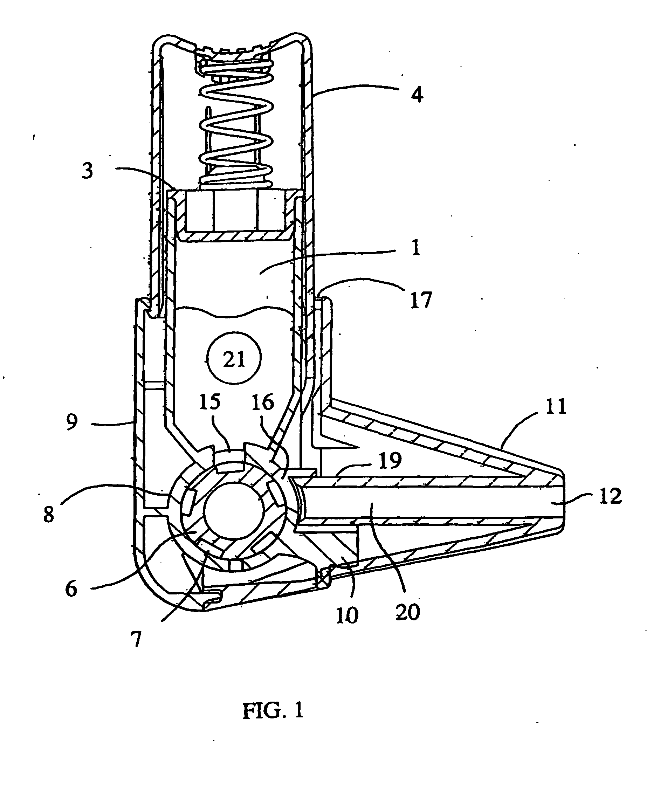

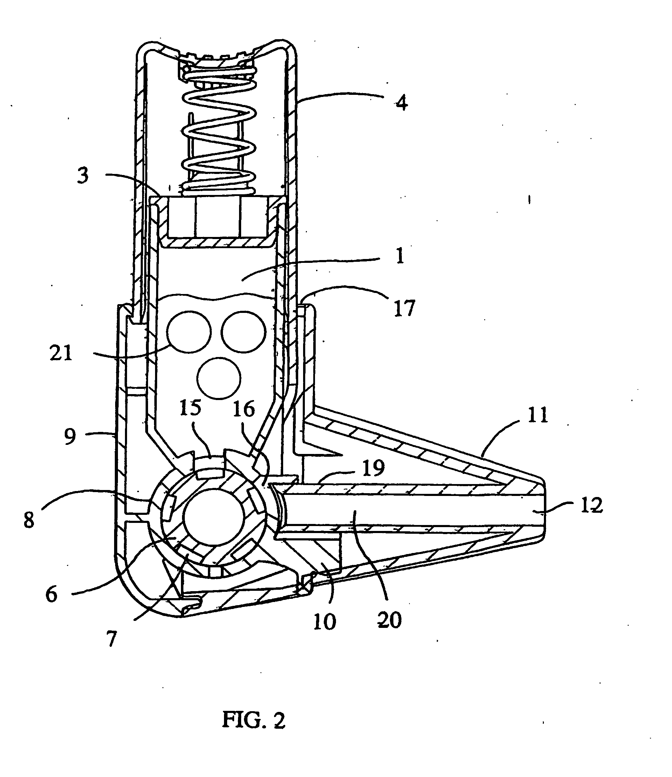

The device of the invention is further illustrated below by way of examples, with reference to Figures 1 and 2.

Fig. 1 shows one embodiment of the device of the invention in a cross-sectional view. The medicament container (1) is generally cylindrical and has a conical end portion with an outlet. Typically, the container (1) has a supply of medicament for e.g. 200 doses. A loose agitation sphere (21) is disposed inside the medicament container (1) such that it is freely movable, when the device is shaken in the flow direction of the powder, i.e. the direction in which the powder is adapted to flow from the container (1) to the dosing recess. A lid (3) closes the upper edge of the medicament container. The depressible cover (4) is adapted to cover the medicament container (1) and the lid (3). A manually rotatable metering drum (6) having five dosing recesses (7) is mounted below the medicament container (1) to the hollow cylindrical body (8), which is moulded together with the medicament container (1).

Moulded together with the medicament container is also the rear wall (9) of the device as well as the projection (10) to receive the mouthpiece (11). The metering drum (6) has, in addition to the dosing recesses, teeth (not shown) which are arranged to engage with the flap extending from the cover (4). The device is actuated by pressing down the cover (4), whereby the flap engage with the teeth causing the metering member rotate so that rotation can only be accomplished stepwise corresponding to the peripheral distance between the dosing recesses (7). The detent drive of the metering drum (6) automatically aligns the dosing recesses (7) with the outlet of the medicament container (1) on the one side and the air channel (20) of the mouthpiece (11) on the other side.

The cylindrical body (8) has an opening (15) through which powder-can fall from the medicament container (1) to the dosing recess when the dosing recess (7) is in alignment with the opening (15). Another opening (16) is provided at the level of the air channel (20) for discharging the powder from the dosing recess (7) to the air channel (20) upon inhalation. In the position shown in Fig. 1 the upper dosing recess is just being filled with the dose of powder from the medicament container (1), while the earlier filled dosing recess has turned to the air channel (20) and is ready to be inhaled. The mouthpiece (11), through which the medical powder can be inhaled, is formed at one side of the inhalation device and has an air channel (20) for distribution of the dose of medicament from the dosing recess (7) into the flow of breathing air. In the area where the mouthpiece is attached, air intakes (17) are provided. The intaken air is led to a slot between the opening (16) of the cylindrical body and a partition wall (19) of the mouthpiece (11). The slot, which is preferably moulded as a nozzle, provides strongly aligned stream of air to the dosing recess (7) blowing the powder out from the dosing recess into the air channel (20) without leaving any residue.

For inhaling a dose of powdered medicament the user first shakes the device in a generally upward and downward motion while maintaining the device in a generally upright position. This results in movement of the agitation sphere in the medicament container and the dosing recess is filled with a dose of medicament. The user then actuates the device by pressing once the cover (4) and then inhales the dose through the mouthpiece.

Fig. 2 shows another embodiment differing from the device of Fig. 1 in that a plurality of agitation spheres (21) are disposed inside the medicament container (1).

Those skilled in the art will recognize that modifications and variations can be made in form and detail to the disclosed embodiments without departing from the spirit and scope of the invention as defined in the following claims. It is considered to be routine for one skilled in the art to make such modifications to the device of the invention.

## Claims

1. A multidose powder inhaler for dispensing powered medicament by inhalation comprising a medicament container (1) adapted to receive a plurality of powdered medicament doses; an air channel (20) though which air is drawn via a mouthpiece (11); a metering member for metering a metered dose of the powdered medicament from the medicament container (1); **characterised by** a loose agitation element (21) disposed inside the medicament container (1).

2. An inhaler according to claim 1, wherein the agitation element (21) is adapted to freely move within the medicament container (1) when the inhaler is shaken.

3. An inhaler according to claim 1 or 2, wherein the agitation element is an agitation sphere (21).

4. An inhaler according to claim 1 or 2, wherein the agitation element is a plurality of agitation spheres (21).

5. An inhaler according to any of claims 1 to 4, wherein the agitation element (21) is made from a conductive material.

6. An inhaler according to claim 5, wherein the agitation element (21) is made of metal or conductive polymer.

7. An inhaler according to any of claims 1 to 6, wherein the charges present in the powder are transferred via the agitation element (21) out of the medicament container (1).

8. An inhaler according to any of claims 3 to 7, wherein the agitation sphere (21) has a rough surface.

9. An inhaler according to claim 1, wherein the metering member has one or more dosing recesses (7) for metering in one position a metered dose of the powdered medicament from the medicament container (1) and for bringing it in another position to the air channel (20).

10. An inhaler according to claim 9, wherein the agitation element (21) has a weight selected to put the necessary surplus weight on the powder, when the device is shaken, such that the filling of the dosing recess (7) is complete.

## Patentansprüche

1. Mehrdosenpulverinhalator zum Verteilen eines pulverisierten Medikaments durch Inhalieren mit
einem Medikamentenbehälter (1), der zur Aufnahme einer Vielzahl von pulverisierten Medikamentendosen ausgelegt ist;
einem Luftkanal (20), durch welchen Luft über ein Mundstück (11) angesaugt wird;
einem Dosierteil zum Dosieren einer dosierten Menge des pulverisierten Medikaments von dem Medikamentenbehälter (1);
**gekennzeichnet durch**
ein loses Rührteil (21), welches in dem Medikamentenbehälter (1) angeordnet ist.

2. Inhalator nach Anspruch 1, in welchem das Rührteil (21) dafür ausgelegt ist, dass es sich in dem Medikamentenbehälter (1) frei bewegt, wenn der Inhalator geschüttelt wird.

3. Inhalator nach Anspruch 1 oder 2, in welchem das Rührteil eine Rührkugel (21) ist.

4. Inhalator nach Anspruch 1 oder 2, in welchem das Rührteil eine Vielzahl von Rührkugeln (21) ist.

5. Inhalator nach einem der Ansprüche 1 bis 4, in welchem das Rührteil (21) aus einem leitfähigen Material hergestellt ist.

6. Inhalator nach Anspruch 5, in welchem das Rührteil (21) aus Metall oder aus leitfähigem Polymer hergestellt ist.

7. Inhalator nach einem der Ansprüche 1 bis 6, in welchem die in dem Pulver vorhandenen Ladungen über das Rührteil (21) aus dem Medikamentenbehälter (1) transportiert werden.

8. Inhalator nach einem der Ansprüche 3 bis 7, in welchem die Rührkugel (21) eine grobe Oberfläche aufweist.

9. Inhalator nach Anspruch 1, in welchem das Dosierteil eine oder mehrere Dosieraussparungen (7) aufweist, um in einer Position eine dosierte Menge des pulverisierten Medikaments aus dem Medikamentenbehälter (1) zu dosieren und um in einer anderen Position diese zu dem Luftkanal (20) zu bringen.

10. Inhalator nach Anspruch 9, in welchem das Rührteil (21) ein Gewicht hat, das derart ausgewählt worden ist, dass das notwendige Mehrgewicht auf das Pulver aufgebracht wird, wenn die Vorrichtung geschüttelt wird, so dass das Befüllen der Dosieraussparung (7) fertiggestellt wird.

## Revendications

1. Inhalateur de poudre à doses multiples pour l'administration d'un médicament en poudre par inhalation, comprenant un récipient à médicament (1) prévu pour recevoir une pluralité de doses de médicament ; un canal d'air (20) à travers lequel de l'air est aspiré via un embout (11) ; un élément de dosage pour doser une dose mesurée du médicament en poudre provenant du réceptacle à médicament (1) ; **caractérisé par** un élément d'agitation (21) libre disposé à l'intérieur du récipient à médicament (1).

2. Inhalateur selon la revendication 1, dans lequel l'élément d'agitation (21) est prévu pour se déplacer librement à l'intérieur du récipient à médicament (1) lorsque l'inhalateur est secoué.

3. Inhalateur selon la revendication 1 ou 2, dans lequel l'élément d'agitation est une sphère d'agitation (21).

4. Inhalateur selon la revendication 1 ou 2, dans lequel l'élément d'agitation comprend plusieurs sphères d'agitation (21).

5. Inhalateur selon l'une quelconque des revendications 1 à 4, dans lequel l'élément d'agitation (21) est en matériau conducteur.

6. Inhalateur selon la revendication 5, dans lequel l'élément d'agitation (21) est en métal ou en polymère conducteur.

7. Inhalateur selon l'une quelconque des revendications 1 à 6, dans lequel les charges présentes dans la poudre sont transférées via l'élément d'agitation (21) hors du récipient à médicament (1).

8. Inhalateur selon l'une quelconque des revendications 3 à 7, dans lequel la sphère d'agitation (21) a une surface rugueuse.

9. Inhalateur selon la revendications 1, dans lequel l'élément de dosage comprend un ou plusieurs alvéoles de dosage (7) pour mesurer dans une position une dose mesurée du médicament en poudre provenant du récipient à médicament (1) et pour l'amener à une autre position vers le canal d'air (20).

10. Inhalateur selon la revendication 9, dans lequel l'élément d'agitation (21) a un poids qui est choisi pour ajouter le surpoids nécessaire sur la poudre, lorsque le dispositif est secoué, de sorte que le remplissage des alvéoles de dosage (7) soit complet.
